# EUROPÄISCHE PATENTSCHRIFT

(11) **EP 0 874 054 B1**
(45) Veröffentlichungstag und Bekanntmachung des Hinweises auf die Patenterteilung: **13.08.2003**
(21) Anmeldenummer: 98106176.5
(22) Anmeldetag: 03.04.1998
(51) Int. Cl.: C12N 15/62, C12N 15/85, C12N 15/13, C12N 15/27, C07K 14/535, C07K 16/42, C07K 16/46, C12N 5/10

(54) **Vektor zur Expression von Immunglobulin-zytokin-Fusionsproteinen in malignen-B-Zellen**
Vector for the expression of immunoglobulin-cytokine fusionproteins in malignant B Cells
Vecteur pour l'expression des protéines du fusion immunoglobuline-cytokine dans les cellules B malignes

(30) Priorität: 22.04.1997 DE 19716892
(43) Veröffentlichungstag der Anmeldung: 28.10.1998
(73) Patentinhaber: GSF-Forschungszentrum für Umwelt und Gesundheit GmbH, 85764 Oberschleissheim (DE)
(72) Erfinder: Mocikat, Ralf, 81369 München (DE)
(74) Vertreter: Reinhard - Skuhra - Weise & Partner

(56) Entgegenhaltungen:
- WO-A-92/08495
- WO-A-92/19266
- WO-A-94/08601
- DE-C- 4 406 512
- SELMAYR MICHAEL ET AL: "B-cell lymphoma idiotypes chimerized by gene targeting can induce tumor immunity." CANCER GENE THERAPY, Bd. 7, Nr. 3, März 2000 (2000-03), Seiten 501-506, XP000972429 ISSN: 0929-1903
- ELKINS K L ET AL: "IN VIVO DELIVERY OF INTERLEUKIN-4 BY A RECOMBINANT VACCINIA VIRUS PREVENTS TUMOR DEVELOPMENT IN MICE" HUMAN GENE THERAPY,XX,XX, Bd. 5, Nr. 7, 1. Juli 1994 (1994-07-01), Seiten 809-820, XP000562626 ISSN: 1043-0342
- WHITMAN ERIC D ET AL: "In vitro and in vivo kinetics of recombinant vaccinia virus cancer-gene therapy." SURGERY (ST LOUIS), Bd. 116, Nr. 2, 1994, Seiten 183-188, XP000972509 ISSN: 0039-6060
- OGGIONI MARCO R ET AL: "Immunization of mice by oral colonization with live recombinant commensal streptococci." VACCINE, Bd. 13, Nr. 8, 1995, Seiten 775-779, XP002157075 ISSN: 0264-410X
- MOCIKAT R: "Improving the expression of chimeric antibodies following homologous recombination in hybridoma cells" JOURNAL OF IMMUNOLOGICAL METHODS,NL,ELSEVIER SCIENCE PUBLISHERS B.V.,AMSTERDAM, Bd. 225, Nr. 1-2, 27. Mai 1999 (1999-05-27), Seiten 185-189, XP004166770 ISSN: 0022-1759
- POZZI G ET AL: "DELIVERY AND EXPRESSION OF A HETEROLOGOUS ANTIGEN ON THE SURFACE OF STREPTOCOCCI" INFECTION AND IMMUNITY,US,AMERICAN SOCIETY FOR MICROBIOLOGY. WASHINGTON, Bd. 60, Nr. 5, 1. Mai 1992 (1992-05-01), Seiten 1902-1907, XP000645180 ISSN: 0019-9567
- LEE S S ET AL: "INTRAVESICAL GENE THERAPY: IN VIVO GENE TRANSFER USING RECOMBINANT VACCINIA VIRUS VECTORS" CANCER RESEARCH,US,AMERICAN ASSOCIATION FOR CANCER RESEARCH, BALTIMORE, MD, Bd. 54, Nr. 13, 1. Juli 1994 (1994-07-01), Seiten 3325-3328, XP000455241 ISSN: 0008-5472

## Beschreibung

Die vorliegende Erfindung betrifft einen Vektor zur Expression von Immunglobulin-Zytokin-Fusionsproteinen in malignen B-Zellen, ein Verfahren zur Expression von Immununglobulin-Zytokin-Fusionsproteinen in malignen B-Zellen, Verwendungen des Vektors sowie maligne B-Zellen, die einen derartigen Vektor enthalten.

Der Immunglobulin-Idiotyp, der auf B-Zell-Lymphomen exprimiert wird, ist ein Tumor-spezifisches Antigen, das jedoch eine geringe Immunogenität im Tumor-tragenden Wirt aufweist. Verschiedene Ansätze sind evaluiert worden, um eine Immunantwort gegen den Idiotyp zu induzieren. U.a. wurde der Idiotyp an GM-CSF gekoppelt und als lösliches Protein zur Vakzinierung von Mäusen verwendet (Nature 362, 755-758, 1993). GM-CSF rekrutiert professionelle Antigen-präsentierende Zellen und führt zur effektiven Präsentation des Idiotyps und damit zur Aktivierung von T-Zellen. Dieser Ansatz hat den Nachteil, daß die Immunglobulin-V-Gene aus dem Lymphom kloniert werden müssen und daß das Fusionsprotein in vitro produziert und gereinigt werden muß. In einer klinischen Situation müßte also für jeden Patienten eine individuelle Vakzine hergestellt werden.

Es ist eine Aufgabe der vorliegenden Erfindung, neue Vektoren bereitzustellen, die in Patienten universell einsetzbar sind, ohne daß individuelle Vakzinen hergestellt werden müssen.

Diese Aufgabe wird erfindungsgemäß gelöst durch einen Vektor zur Expression von Immunglobulin-Zytokin-Fusionsproteinen in malignen B-Zellen, mindestens enthaltend, in funktioneller Verbindung,
a) einen Bereich von mindestens 1,5 kb, der homolog zu einem Bereich des µ-Introns oder κ-Introns ist, der wahlweise einen oder keinen oder keinen funktionellen C_{µ}- oder C_{κ}-Enhancer enthält;
b) mindestens eine DNA-Sequenz, die eine Domäne eines Immunglobulins oder eines Teils hiervon codiert;
c) eine für ein Zytokin codierende DNA-Sequenz; und
d) einen in eukaryontischen B-Zellen selektierbaren Marker, der wahlweise einen Enhancer enthält oder der keinen funktionellen Enhancer-Bereich aufweist, wobei dann die Expression dieses Markers nach der Integration vom zellulären C_{µ}- oder C_{κ}-Enhancer gesteuert wird.

Bevorzugte Ausgestaltungen der Erfindung ergeben sich aus den Unteransprüchen, der nachfolgenden Beschreibung sowie dem Ausführungsbeispiel.

Während im Stand der Technik, beispielsweise in der oben zitierten Veröffentlichung in Nature 362, 755-758, 1993, die erhaltenen Fusionsproteine nach Transfektion von Zellkulturzellen in vitro exprimiert, gereinigt und in löslicher Form verabreicht wurden, kann durch die erfindungsgemäß bereitgestellten Vektoren das Zytokingen durch homologe Rekombination in den Schwere-Ketten-Locus von Immunglobulingenen stellenspezifisch eingebaut werden, ohne daß das V-Gen isoliert und in den Vektor eingebaut werden müßte. Der erfindungsgemäße Vektor wird direkt in die maligne B-Zelle eingebaut und in dieser Zelle zur Expression gebracht, sodaß anstatt wie bei bisherigen Ansätzen nicht das lösliche, vorher gereinigte Protein verabreicht werden muß, sondern die gentechnisch veränderten Tumorzellen direkt an den Patienten verabreichbar sind. Dieser bringt zum einen eine große Zeit-, Arbeits- und Kostenersparnis und hat zum anderen auch einen wesentlich besseren Tumor-protektiven Effekt zur Folge.

Ausgangspunkt zur Konstruktion der erfindungsgemäßen Vektoren waren die in der DE 44 06 512 beschriebenen Integrationsvektoren zur Herstellung rekombinanter Antikörper. Diese Vektoren eignen sich zu einer hoch-effizienten Produktion von chimären Maus /Mensch-Antikörpern. Die in der DE 44 06 512 beschriebenen Integrationsvektoren dienten lediglich zur Herstellung von rekombinanten Antikörpern und wurden deshalb ausschließlich in Antikörper produzierenden Hybridomzellen zur Expression gebracht. Der erfindungsgemäße Lösungsansatz, Immunglobuline als Fusionsproteine mit Zytokinen durch homologe Rekombination direkt in malignen B-Zellen zur Expression zu bringen und derart modifizierte maligne B-Zellen zur Vakzinierung von Patienten mit malignen B-Zell-Tumoren zu verwenden, wird durch die DE 44 06 512 nicht nahegelegt.

Durch die vorliegende Erfindung wird ein Zytokingen in den Immunglobulin-Schwere-Ketten-Lokus maligner B-Zellen, beispielsweise B-Zell-Lymphom-Zellen, mittels homologer Rekombination eingeführt. Hierzu wurde ein Integratiosvektor mit den Merkmalen des Anspruchs 1 konstruiert. Nach stellenspezifischer Integration in den Schwere-Ketten-Lokus wird unter der Kontrolle des endogenen V_{H}-Promotors ein Immunglobulin-Zytokin-Fusionsprotein exprimiert.

Der beschriebene Rekombiationsvektor ist potentiell für alle Lymphome einsetzbar. Durch die Einführung des Zytokingens durch homologe Rekombination in den Schwere-Ketten-Lokus der malignen B-Zelle kann eine Isolierung des Idiotyps vermieden werden. Die Zeit bis zum Beginn einer Therapie wird dadurch erheblich verkürzt. Weiterhin ist es möglich, die durch homologe Rekombination modifizierten Tumorzellen nach Bestrahlung für die Vakzinierung einzusetzen, ohne daß das Fusionsprotein gereinigt werden muß. Das nachfolgende Ausführungsbeispiel zeigt, daß der Tumor-protektive Effekt wesentlich ausgeprägter ist, wenn mit den erfindungsgemäß bereitgestellten, modifizierten Zellen anstatt mit dem löslichen Protein immunisiert wird.

Grundlage für die Konstruktion des erfindungsgemäßen Vektors sind die in der DE 44 06 512 beschriebenen Integrationsvektoren. Auf diese Deutsche Patentschrift wird zur vollständigen Offenbarung voll inhaltlich Bezug genommen. Allerdings werden die in der DE 44 06 512 beschriebenen Integrationsvektoren erfindungsgemäß derart modifiziert, daß keine rekombinanten Antikörper exprimiert und gewonnen werden, sondern Fusionsproteine aus Immunglobulinen und Zytokinen, die direkt in malignen B-Zellen zur Expression gebracht werden.

Der erfindungsgemäße Vektor kann einen zu einem Intron-Bereich homologen Bereich von mindestens 1,5 kb aufweisen, in den der Ig-Enhancer natürlicherweise nicht vorkommt oder aus dem er deletiert oder in dem er inaktiviert wurde; oder in einer weiteren Ausführungsform weist der erfindungsgemäße Vektor aber einen funktionellen C_{µ}- oder C_{κ}-Enhancer auf.

Die DNA kann beispielsweise ein oder mehrere Exons umfassen, solange eine funktionelle Domäne eines Antikörpers oder ein funktioneller Teil davon codiert wird. Ist der funktionelle Teil der Domäne Teil einer V-Domäne, so muß diese zur Bindung an das Zielantigen fähig sein oder dazu beitragen. Handelt es sich um einen Teil einer C-Domäne, so muß diese mindestens einen Teil der Effektorfunktionen ausüben können.

In einer bevorzugten Ausführungsform umfaßt der Bereich des µoder κ-Introns von mindestens 1,5 kb den Bereich, in dem der C_{µ}- oder C_{κ}-Enhancer lokalisiert ist, wobei dieser Bereich wahlweise einen oder keinen funktionellen C_{µ}- oder C_{κ}-Enhancer mehr enthält.
Der Enhancer kann in dieser zuletzt genannten Ausführungsform entweder deletiert oder inaktiviert sein. Eine solche Inaktivierung kann z. B. durch Mutagenese nach im Stand der Technik benannten Verfahren herbeigeführt werden.

Bei dem selektierbaren Marker ist der Enhancer deletiert oder inaktiviert worden. In einer anderen Ausführungsform weist der Marker keinen natürlichen Enhancer auf. In einer weiteren Ausführungsforn besitzt der selektierbare Marker einen Enhancer.

Die im Vektor enthaltene homologe Sequenz muß eine Länge von mindestens 1,5 kb aufweisen, um ein homologes Rekombinationsereignis überhaupt erzielen zu können. Diese DNA-Sequenz von mindestens 1,5 kb kann aus verschiedenen Bereichen des C_{µ}- oder C_{κ}-Introns gewählt werden. Der Enhancer selbst kann erfindungsgemäß nicht im Konstrukt enthalten sein oder aus der Homologieflanke deletiert bzw. darin inaktiviert worden sein. Bei der Integration des Vektors in die homologe Sequenz eines funktionell rearrangierten Antikörpergens wird die Expression des rekombinanten Gens unter die Kontrolle des endogenen Enhancers gestellt. Werden Exons, die konstante Regionen codieren, einrekombiniert, so stehen diese ferner unter der Kontrolle des endogenen V-Promotors. Der Enhancer reguliert darüber hinaus die Expression des selektierbaren Markers. Dadurch wird sichergestellt, daß der Selektionsmarker nur dann aktiviert wird, wenn er in der Nähe eines starken Enhancers integriert wird. Durch die verwendete Homologieflanke wird eine homologe Rekombination mit dem Immunglobulinlocus begünstigt, wodurch der selektierbare Marker unter die Kontrolle des endogenen C_{µ}- oder C_{κ}-Enhancers gestellt wird.

Die Expression des erfindungsgemäß bereitgestellten rekombinanten Gens, das für ein Immunglogbulin-Zytokin-Fusionsprotein codiert, wird nach stellenspezifischer Integration 3' vom Schwere-Ketten-V-Gen der malignen B-Zelle vom endogenen V_{H}-Promotor gesteuert.

Klonierungstechniken sind dem Fachmann aus dem Stand der Technik bekannt. Beispielsweise wird verwiesen auf Sambrook et al, "Molecular Cloning, A Laboratory Manual", 2. Auflage 1989, Cold Spring Harbor Laboratory, Cold Spring Harbor, USA, sowie Harlow und Lane "Antibodies, A Laboratory Manual" 1988, Cold Spring Harbor Laboratory, Cold Spring Harbor, USA.

Das endogene Immunglobulin-V-Gensegment der Immunglobulin-produzierenden B-Zelle bleibt unverändert und wird nach der homologen Rekombination im Zusammenhang mit den eingeführten konstanten Gensegmenten sowie dem Zytokinen exprimiert. Auf diese Weise bleibt der Idiotyp eines B-Zell-Lymphoms erhalten, seine physikalische Bindung an das Zytokin führt jedoch zu einer verstärkten Präsentation durch Antigen-präsentierende Zellen und damit zu einer erhöhten Immunogenität des Isotyps. Der erfindungsgemäße Vektor codiert also im Gegensatz zum Vektor der DE 44 06 512 keine V-Domäne oder einen Teil hiervon; stattdessen bleibt die endogene V-Sequenz und der Idiotyp der transfizierten B-Zelle erhalten.

In einer anderen bevorzugten Ausführungsform weist der Vektor eine DNA-Sequenz auf, die eine konstante Region oder einen Teil davon codiert. Diese Sequenz kann entweder für die schwere oder leichte Kette eine Antikörpers codieren. Dem Fachmann ist bekannt, daß bei allen Isotypen mehrere Exons für die schwere Kette codieren. Sofern nur ein C_{H}-Exon für die schwere Kette im Konstrukt vorhanden ist, ist dies vorzugsweise das C_{H1}-Exon. Mit einem derartigen Konstrukt können Fusionsproteine hergestellt werden, deren Immunglobulinanteil die Funktionalität von Faboder F(ab)₂-Fragmenten aufweisen. Vorzugsweise enthält der erfindungsgemäße Vektor jedoch sämtliche Exons eines schwere Kette-Isotyps, wodurch eine vollständige schwere Kette exprimiert werden kann. In dieser Ausführungsform sind die Elemente (a), (b), (c) und (d) in dieser Reihenfolge aufeinanderfolgend in 5'-3'-Richtung angeordnet.

In einer andereren bevorzugten Ausführungsform des erfindungsgemäßen Integrationsvektors weist der zum p- oder K-Intron homologe Bereich eine Länge von 1,9 kb oder 2,0 kb auf.

In einer weiteren bevorzugten Ausführungsform enthält der erfindungsgemäße Vektor eine Bakterien-kompatible Regulationseinheit. Eine derartige Bakterien-kompatible Regulationseinheit ermöglicht die Klonierung und Amplifizierung des Vektors in bakteriellen Systemen, beispielsweise in E. coli. Bakterienkompatible Regulationssysteme sind dem Fachmann aus dem Stand der Technik bekannt; vgl. Sambrook et al, a.a.O. Ein Beispiel für eine derartige bakterienkompatible Regulationseinheit ist die Regulationseinheit aus dem Plasmid pBR322.

In einer weiteren Ausführungsform ist der Immunglobulinanteil des Fusionsproteins chimärer Natur. Unter "chimär" wird ein Immunglobulin verstanden, das die V- und C-Regionen aus verschiedenen Spezies kombiniert. Beispielsweise kann ein V-Gen aus der Maus mit den C-Exons eines menschlichen Isotyps kombiniert werden.

In einer weiteren Ausführungsform codiert die DNA-Sequenz des Merkmals (b) eine menschliche Immunglobulinkette. Diese Domäne können sowohl V- alsauch C-Domänen oder Teile davon sein.

In einer weiteren bevorzugten Ausführungsform des erfindungsgemäßen Vektors codiert die DNA-Sequenz Domänen, die aus der Maus, Ratte, Ziege, aus dem Pferd oder Schaf stammen. Vorzugsweise stammen sämtliche DNA-Sequenzen für entweder die V- oder die C-Regionen codierenden Sequenzen aus einer dieser Tierarten, wobei die C-Regionen auch aus verschiedenen Tierarten entnehmbar sind.

In einer vorteilhaften Ausführungsform der vorliegenden Erfindung weist der Vektor eine für den Patienten xenogene konstante Ig-Region auf, was sich für die Induktion einer Immunantwort als vorteilhaft erweisen kann.

Wie auch die in einer anderen Ausführungsform des erfindungsgemäßen Vektors verwendeten menschliche Domänen codierenden DNA-Sequenzen können diese Domänen codierende DNA-Sequenzen für die homologe Rekombination mit der entsprechenden Sequenz aus einer anderen Säugerart eingesetzt werden.

In einer anderen bevorzugten Ausführungsform des erfindungsgemäßen Vektors codiert die DNA-Sequenz sämtliche C-Domänen eines sekretorischen Antikörpers.

Eine weitere bevorzugte Ausfürungsform des erfindungsgemäßen Vektors enthält eine DNA-Sequenz, die C-Domänen eines Antikörpers codiert, der IgM-, IgG1-, IgG2a-, IgG2b-, IgG4-, IgA-, IgD- oder IgE-Antikörper ist. Dem Fachmann ist bekannt, daß manche dieser Isotypen nicht in allen Säugerspezies vorkommen. So enthält das menschliche Genom beispielsweise C-Gene, die IgG4-Isotype, nicht aber IgG2a- oder IgG2b-Isotype codieren. Dagegen enthält das Maus-Genom C-Gene, die den IgG2a- und den IgG2b-Isotyp, nicht aber den IgG4-Isotyp codieren.

In einer weiteren bevorzugten Ausführungsform des erfindungsgemäßen Integrationsvektors ist der selektierbare Marker gpt, neo oder codiert für Hygromycin-Resistenz. Der Fachmann ist mit der Züchtung von Zellen unter Selektionsbedingungen, die diese Marker erfordern, vertraut (vgl. z. B. Sambrook, et al, a.a.O.). Er ist darüber hinaus in der Lage, weitere Selektionsmarker auszuwählen, die im erfindungsgemäßen Vektor verwendet werden können.

Die Transfektion Immunglobulin-produzierender Zellen gilt als Standardverfahren der modernen Immunologie. Dem Fachmann ist bekannt, daß die Transfektionsbedingungen für jede verwendete Zellinie eingestellt werden müssen. Ein Leitfaden zur Errichtung solcher Transfektionsbedingungen ist beispielsweise in Toneguzzo et al, Mol. Cell Biol. 6 (1986), 703-706 sowie in der Beschreibung zum Biorad "Genepulser" gegeben. Für die Mausmyelomlinie NS-1 (ATCC TIB 18) beispielsweise sind geeignete Transfektionsbedingungen in Mocikat et al, Gene 136 (1993), 349-353 beschrieben. Die Selektion von stabilen Transformanten erfolgt durch Züchtung der Transformanten für mindestens 7 Tage in einem geeigneten Selektionsmedium. Die Selektion stabiler Transformanten ist notwendig, um Zellen abzutöten, die das Plasmid nicht aufgenommen haben. Die Wahl des Selektionsmediums richtet sich selbstverständlich nach dem verwendeten Selektionsmarker. Die Herstellung geeigneter Selektionsmedien ist im Stand der Technik bekannt und beispielsweise in Sanbrook et al, a.a.O. nachlesbar.

In einer bevorzugten Ausführungsform des erfindungsgemäßen Verfahrens erfolgt die Transfektion durch Elektroporation, Calcium-Kopräzipitation, Lipofektion, die DEAE-Dextran-Technik oder retroviralen Gentransfer. All diese Verfahren sind im Stand der Technik gut bekannt. Der Fachmann weiß daher, wie er die Bedingungen für die einzelnen Transfektionsverfahren im erfindungsgemäßen Verfahren einzustellen hat. In einer anderen bevorzugten Ausführungsform des erfindungsgemäßen Verfahrens erfolgt die Selektion in einem Medium, das als Selektionsmittel Mykophenolsäure, G418, oder Hygromycin enthält. Wie bereits vorstehend erwähnt, sind diese Selektionsmittel im Stand der Technik gut bekannt. Ihre Wahl hängt vom verwendeten Selektionsmarker ab, während ihre Dosierung aus Standardwerken der Molekularbiologie hergeleitet werden kann; vgl. z. B. Sambrook et al., a.a.O.
In einer anderen bevorzugten Ausführungsform des erfindungsgemäßen Verfahrens codiert die DNA-Sequenz konstante Domänen des γ₁-, γ₂ₐ-, γ_{2b}-, γ₃-, γ₄-, µ-, α-, δ-, oder ∈-Isotyps.

Die DNA-Sequenz gemäß Merkmal (c) codiert für Zytokine, die ausgewählt werden aus Interleukinen, Interferonen, Kolonie-stimulierenden Faktoren, Lymphokinen und Wachstumsfaktoren. Beispiele für derartige DNA-Sequenzen sind: IL-2, IL-4, IL-7, IL-12, IL-13, GM-CSF oder Interferon γ.

Die Vektoren der vorliegenden Erfindung werden beispielsweise durch die oben näher bezeichneten Verfahren in maligne B-Zellen eingebracht und dort durch homologe Rekombination integriert und stabil exprimiert. Durch geeignete Selektions- und Identifizierungsverfahren werden derartige maligne B-Zellen identifiziert, die das Fusionsprotein stabil exprimieren. In einer Ausführungsform der vorliegenden Erfindung ist es jedoch auch möglich, maligne B-Zellen, die das erfindungsgemäße Vektorkonstrukt enthalten, direkt zur Vakzinierung einzusetzen, ohne daß eine vorherige Selektion auf solche Zellen stattgefunden hat, die das Fusionsprotein stabil exprimieren. Selbstverständlich ist es vor einer Vakzinierung notwendig, die malignen B-Zellen vor der Injektion durch Bestrahlung replikationsinkompentent zu machen, ohne daß hierdurch die Expression des Fusionsproteins beeinträchtigt würde.

Somit ist es nicht zwingend notwendig, eine Selektion der rekombinanten Zellen vor der Injektion in den Patienten vorzunehmen. Für die Tumorimmunisierung ist die Expression der Transformanten, in denen ein homologes Rekombinationsereignis vorliegt, ausreichend, so daß durchaus auch eine heterogene Zellpopulation verabreicht werden kann. Hierdurch ist es nicht zwingend notwendig, daß der erfindungsgemäße Vektor den im Merkmal (d) des Anspruchs 1 genannten, in eucaryontischen B-Zellen selektierbaren Marker aufweist. Auf einen derartigen Marker kann in solchen Fällen, bei denen keine Selektion der rekombinanten Zellen vor der Injektion in den Patienten stattfindet, verzichtet werden.

Der erfindungsgemäß bereitgestellte Vektor ist auch in einem ex vivo-Ansatz einsetzbar. Hierzu werden kultivierte, dendritische Zellen mittels des von den rekombinierten Tumorzellen exprimierten Fusionsproteins zur Präsentation tumorspezifischer Peptide und eventuell auch zur Aktivierung von T-Zellen gebracht. Die Antigen-präsentierenden Zellen bzw. die aktivierten T-Zellen würden dann in den Patienten zurückgegeben werden.

Ein großer Vorteil bei der Injektion derartiger maligner B-Zellen, die das Immunglobulin-Zytokin-Fusionprotein produzieren, liegt im Wegfall von aufwendigen Produktions- und Reinigungsschritten zur Herstellung dieser Proteine in klinikrelevanten Mengen.

Die maligne B-Zelle, in die der erfindungsgemäße Vektor eingebracht wird, kann beispielsweise eine B-Zell-Leukämie-Zelle, eine B-Zell-Lymphom-Zelle oder eine Plasmazytomzelle sein.

Die Erfindung wird nachstehend anhand eines an einem Tiermodell durchgeführten Beispiels beschrieben. Die hierdurch gewonnenen Ergebnisse sind aufgrund lang andauernder Erfahrungen auch auf den Menschen übertragbar. Selbstverständlich ist die Erfindung nicht auf das nachfolgende spezielle Ausführungsbeispiel beschränkt, sondern kann im Rahmen der nachfolgenden Ansprüche abgewandelt werden.

### Beispiel:

### Vektorkonstruktion

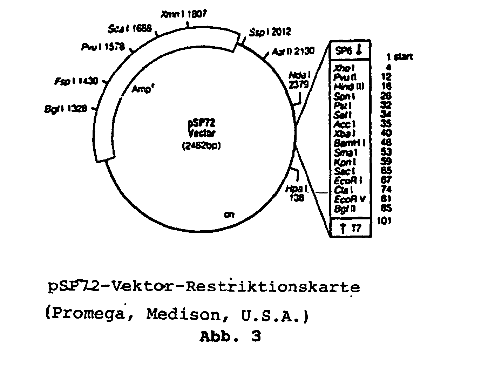

Das murine GM-CSF-Gen wird über PstI in pSP72 (vgl. Abb. 3) einkloniert. Der 5'-Anteil des Gens wird mit EcoRV und XmaI exzidiert und durch ein ebenso geschnittenes PCR-Produkt ersetzt, dem die N-terminalen 29 Aminosäuren fehlen. Auf diese Weise entsteht das Konstrukt pSP72 (ΔEV)-GMCSF (ΔL). In den Vekor pSVgpt-huγ1-A5 (Kardinal et al. Eur. J. Immunol. 25, 792-797, 1995) wird 3' des humanen IgG1-C_{H}3-Exons eine SalI-Schnittstelle eingeführt. Das GM-CSF-Gen wird mit SalI aus pSP72 (ΔEV)-GMCSF (ΔL) herausgeschnitten und in die SalI-Stelle des modifizierten psVgpt-huγ1-A5 ligiert.

### Gentransfer

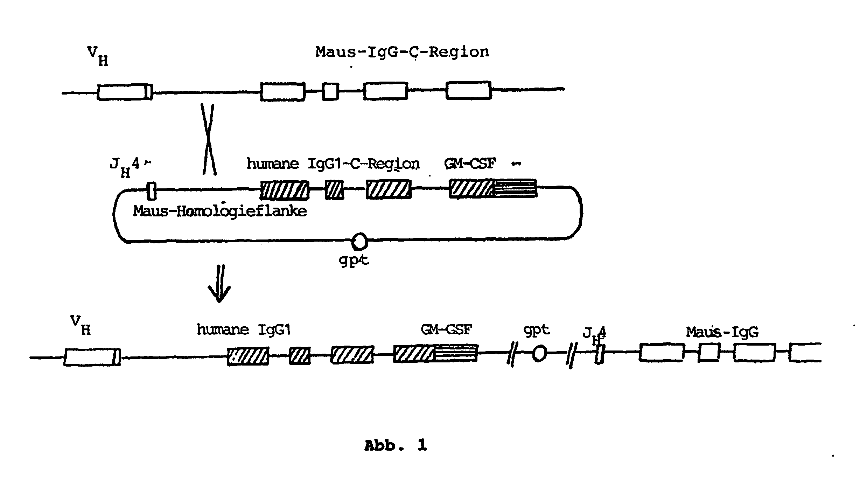

Der GM-CSF-tragende Rekombinationsvektor wird mit EcoRI oder BamHI linearisiert und mittels Elektroporation in die murine B-Zell-Lymphom-zellinie MPC11 transferiert. Stabil transfizierte Zellen werden mit Hilfe von Mykophenolsäure auf die Anwesenheit des gpt-Gens selektioniert. Die Klone, in denen das Konstrukt stellenspezifisch integriert wurde (s. Abb. 1), werden im ELISA identifiziert.

### Tierversuche

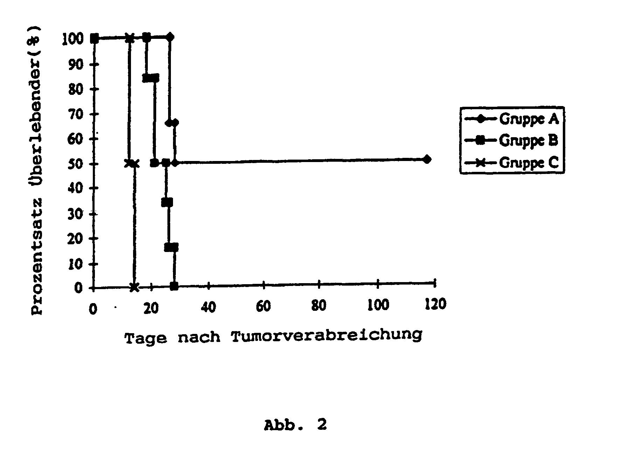

Der Nutzen, der die Modifikation eines Lymphom-Idiotyps durch homologe Rekombination für die Tumorimmunisierung bringt, wird am Beispiel des murinen B-Zell-Lymphoms MPC11 gezeigt. Dieser Tumor stammt von der BALB/c-Maus ab, exprimiert IgG2b und führt nach Inokulation von 10³ Zellen in syngene Tiere innerhalb von bis zu 20 Tagen bei 100% der Tiere zum Tod. Nach Transfer des Vektors in MPC11 und der Identifizierung von homologen Rekombinanten werden diese für die Immunisierung von BALB/c-Mäusen eingesetzt. Dazu werden je 5 x 10⁶ bestrahlte Zellen zweimal im Abstand von drei Wochen i.p. injiziert. 7 Tage nach der letzten Injektion wird ein letales Inokulum von Wildtyp-Tumorzellen i.p. verabreicht . Während die Tiere der Kontrollgruppe, die nur Tumorzellen, aber keine Präimmunisierung erhalten haben, am Tumor sterben (Gruppe C in Abb. 2), zeigen die immunisierten Tiere einen signifikanten Überlebensvorteil (Gruppe A). Wenn mit MPC11-Zellen vakziniert wird, in deren Schwere-Ketten-Lokus lediglich die humane IgGl-C-Region ohne das GM-CSF-Gen durch homologe Rekombination eingeführt wurde, die also eine xenogenisierte schwere Kette exprimieren, so kommt nur eine marginale Überlebenszeit-Verlängerung zustande.

Da der Idiotyp des Tumors bei dem beschriebenen Verfahren weder auf genetischer noch auf Protein-Ebene aus dem Lymphom isoliert werden muß und keine individualspezifische Vakzine hergestellt werden muß, sondern ein universeller Vektor für alle Lymphome anwendbar ist, kann die Zeit bis zum Beginn der Therapie erheblich verkürzt werden. Der Einsatz der durch homologe Rekombination modifizierten, bestrahlten Tumorzellen bringt nicht nur den Vorteil, daß auf die zeit- und kostenaufwendige Reinigung des Fusionsproteins verzichtet werden kann. Ein entscheidender Vorteil besteht darin, daß der Tumor-protektive Effekt wesentlich ausgeprägter ist, wenn mit Zellen immunisiert wird, die ein rekombinantes Protein exprimieren, als wenn das gereinigte lösliche Protein gegeben wird.

Durch den vorliegenden Rekombinationsvektor kann die Zeit bis zu Beginn einer Therapie erheblich verkürzt werden. Weiterhin ist es möglich, die durch homologe Rekombination modifizierten Tumorzellen nach Bestrahlung für die Vakzinierung einzusetzen, ohne daß das Fusionsprotein aufgereinigt werden muß. Es konnte durch das obige Beispiel gezeigt werden, daß der Tumor-protektive Effekt überraschenderweise wesentlich ausgeprägter ist als bei Immunisierung der Zellen mit gereinigtem, löslichem Protein.

## Patentansprüche

1. Vektor zur Expression von Immunglobulin-Zytokin-Fusionsproteinen in malignen B-Zellen, mindestens enthaltend, in funktioneller Verbindung,
(a) einen Bereich von mindestens 1,5 kb, der homolog zu einem Bereich des µ-Introns oder κ-Introns ist;
(b) mindestens eine DNA-Sequenz, die eine Domäne eines Immunglobulins oder eines Teils hiervon codiert;
(c) eine für ein Zytokin codierende DNA-Sequenz; und
(d) ein in eukaryontischen B-Zellen selektierbares Markergen, das einen funktionellen einen nicht-funktionellen oder keinen Enhancer-Bereich aufweist.

2. Vektor nach Anspruch 1, wobei der mindestens 1,5 kb große Bereich einen funktionellen C_{µ}- oder C_{κ}-Enhancer enthält.

3. Vektor nach Anspruch 1, wobei der mindestens 1,5 kb große Bereich einen nicht funktionellen C_{µ}- oder C_{κ}-Enhancer enthält.

4. Vektor nach einem oder mehreren der vorhergehenden Ansprüche, wobei das in eukaryontischen B-Zellen selektierbare Markergen einen nicht-funktionellen Enhancer-Bereich aufweist.

5. Vektor nach einem oder mehreren der vorhergehenden Ansprüche, wobei das in eukaryontischen B-Zellen selektierbare Markergen keinen Enhancer-Bereich aufweist.

6. Vektor nach einem oder mehreren der vorhergehenden Ansprüche, wobei die DNA-Sequenz aus (b) eine konstante Region oder einen Teil davon codiert.

7. Vektor nach einem oder mehreren der vorhergehenden Ansprüche, wobei der Bereich, der homolog zu einem den C_{µ}- oder C_{κ}-Enhancer umfassenden Bereich des µ- oder κ-Introns ist, mindestens 1,9 kb umfaßt.

8. Vektor nach einem oder mehreren der vorhergehenden Ansprüche, wobei der Bereich, der homolog zu einem den C_{µ}- oder C_{κ}-Enhancer umfassenden Bereich des µ- oder κ-Introns ist, mindestens 2,0 kb umfaßt.

9. Vektor nach einem oder mehreren der vorhergehenden Ansprüche, der eine Bakterien-kompatible Regulationseinheit enthält.

10. Vektor nach einem oder mehreren der vorhergehenden Ansprüche, wobei das Immunglobulin ein chimäres Immunglobulin ist.

11. Vektor nach einem oder mehreren der vorhergehenden Ansprüche, wobei die DNA-Sequenz aus (b) für die Domäne einer menschlichen Immunglobulinkette codiert.

12. Vektor nach einem oder mehreren der vorhergehenden Ansprüche, wobei die DNA-Sequenz aus (b) Domänen codiert, die aus der Maus, Ratte, Ziege, aus dem Pferd oder Schaf stammen.

13. Vektor nach einem oder mehreren der vorhergehenden Ansprüche, wobei die DNA-Sequenz aus (b) sämtliche C-Domänen eines sekretorischen Antikörpers codiert.

14. Vektor nach einem oder mehreren der vorhergehenden Ansprüche, wobei die DNA-Sequenz aus (b) sämtliche C-Domänen eines membrangebundenen Antikörpers codiert.

15. Vektor nach einem oder mehreren der vorhergehenden Ansprüche, **dadurch gekennzeichnet, daß** die DNA-Sequenz aus (c) für Interleukine, Interferone, Kolonie-stimulierende Faktoren, Lymphokine oder Wachstumsfaktoren codiert.

16. Vektor nach Anspruch 15, **dadurch gekennzeichnet, daß** die DNA-Sequenz aus (c) für IL-2, IL-4, IL-7, IL-12, IL-13, GM-CSF oder Interferon γ codiert.

17. Vektor nach einem oder mehreren der vorhergehenden Ansprüche, wobei der selektierbare Marker gpt, neo, oder ein Hygromycin-Resistenz codierender Marker ist.

18. Verfahren zur Expression eines Immunglobulin-Zytokin-Fusionsproteins in malignen B-Zellen in vitro mit den nachfolgenden Schritten:
(a) Einbringen eines Vektors nach einem oder mehreren der Ansprüche 1-17 in eine maligne B-Zelle;
(b) Selektion und Identifizierung von Zellen, die das Fusionsprotein stabil exprimieren;
(c) Behandeln der Zellen, um sie replikationsinkompetent zu machen.

19. Verfahren nach Anspruch 18, wobei der Schritt (b) entfällt.

20. Verfahren nach Anspruch 18 oder 19, wobei der Schritt (a) durch Transfektion erfolgt.

21. Verfahren nach Anspruch 20, wobei die Transfektion durch Elektroporation, Calciumphosphat-Kopräzipitation, Lipofektion, die DEAE-Dextran-Technik, oder retroviralen Gentransfer erfolgt.

22. Verfahren nach einem oder mehreren der Ansprüche 18 bis 21, wobei die Selektion in einem Medium erfolgt, das als Selektionsmittel Mykophenolsäure, G418 oder Hygromycin enthält.

23. Verfahren nach einem oder mehreren der Ansprüche 18 bis 22, wobei nach dem Einbringen eines Vektors nach einem oder mehreren der Ansprüche 1 bis 17 durch homologe Rekombination eine stellenspezifische Integration des Vektors 3' vom Schwere-Ketten-V-Gen der malignen B-Zelle erfolgt.

24. Verfahren nach einem oder mehreren der Ansprüche 18 bis 23, wobei die Expression vom endogenen V_{H}-Promotor gesteuert wird.

25. Verwendung eines Vektors nach einem oder mehreren der Ansprüche 1 bis 17 zur Expression von Immunglobulin-Zytokin-Fusionsproteinen in malignen B-Zellen.

26. Verwendung nach Anspruch 25, wobei die maligne B-Zelle eine B-Zell-Leukämie-Zelle, eine B-Zell-Lymphom-Zelle oder eine Plasmozytomzelle ist.

27. Verwendung nach Anspruch 25, wobei durch die Expression der Immunglobulin-Zytokin-Fusionsproteine eine Aktivierung von T-Zellen erfolgt.

28. Verwendung eines Vektors nach einem oder mehreren der Ansprüche 1 bis 17 in malignen B-Zellen zur Vakzinierung von Patienten mit malignen B-Zell-Erkrankungen.

29. Maligne B-Zelle, enthaltend einen Vektor nach einem oder mehreren der Ansprüche 1 bis 17 in integrierter Form, wobei von der Zelle ein Immunglobulin-Zytokin-Fusionsprotein exprimiert wird.

30. Verwendung einer malignen B-Zelle, die replikationsinkompetent gemacht wurde und einen Vektor nach einem oder mehreren der Ansprüche 1 bis 17 in integrierter Form enthält und zur Expression eines Immunglobulin-Zytokin-Fusionsproteins befähigt ist, zur Herstellung einer pharmazeutischen Zubereitung zur Behandlung von Patienten mit malignen B-Zell-Erkrankungen.

## Claims

1. Vector for the expression of immunoglobulin-cytokin fusion proteins in malignant B cells, at least containing operably linked to each other
(a) a region of at least 1.5 kb which is homologous to a region of the µ intron or the κ intron;
(b) at least one DNA sequence encoding a domain of an immunoglobulin or a part thereof;
(c) a DNA sequence encoding a cytokin; and
(d) a marker gene which is selectable in eukaryotic B cells and contains a functional or non-functional or no enhancer region.

2. Vector according to claim 1, wherein said region of at least 1.5 kb contains a functional C_{µ} or C_{κ} enhancer.

3. Vector according to claim 1, wherein said region of at least 1.5 kb contains a non-functional C_{µ} or C_{κ} enhancer.

4. Vector according to one or more of the preceding claims, wherein the marker gene selectable in eukaryotic B cells contains a non-functional enhancer region.

5. Vector according to one or more of the preceding claims, wherein the marker gene selectable in eukaryotic B cells lacks an enhancer region.

6. Vector according to one or more of the preceding claims, wherein the DNA sequence of (b) encodes a constant region or a part thereof.

7. Vector according to one or more of the preceding claims, wherein the region homologous to a region comprising the C_{µ} or the C_{κ} enhancer of the µ or the κ intron comprises at least 1.9 kb.

8. Vector according to one or more of the preceding claims, wherein the region homologous to a region comprising the C_{µ} or the C_{κ} enhancer of the µ or the κ intron comprises at least 2.0 kb.

9. Vector according to one or more of the preceding claims, said vector containing a regulatory unit which is compatible with bacteria.

10. Vector according to one or more of the preceding claims, wherein the immunoglobulin is a chimeric immunoglobulin.

11. Vector according to one or more of the preceding claims, wherein the DNA sequence of (b) encodes the domain of a human immunoglobulin chain.

12. Vector according to one or more of the preceding claims, wherein the DNA sequence of (b) encodes domains derived from mouse, rat, goat, horse or sheep.

13. Vector according to one or more of the preceding claims, wherein the DNA sequence of (b) encodes all the C domains of a secretory antibody.

14. Vector according to one or more of the preceding claims, wherein the DNA sequence according to (b) encodes all the C domains of a membrane-bound antibody.

15. Vector according to one or more of the preceding claims, **characterized in that** said DNA sequence of (c) encodes interleukins, interferons, colony-stimulating factors, lymphokins or growth factors.

16. Vector according to claim 15, **characterized in that** said DNA sequence of (c) encodes IL-2, IL-4, IL-7, IL-12, IL-13, GM-CSF or interferon γ.

17. Vector according to one or more of the preceding claims, wherein the selectable marker gene is gpt, neo or a marker gene encoding hygromycin resistance.

18. Method for the expression of an immunoglobulin-cytokin fusion protein in malignant B cells in vitro including the following steps of:
(a) introduction of a vector according to one or more of claims 1 - 17 into a malignant B cell;
(b) selection and identification of cells stably expressing the fusion protein;
(c) treatment of the cells to render them replication-incompetent.

19. Method according to claim 18, wherein step (b) is ommitted.

20. Method according to claims 18 or 19, wherein step (a) is performed by means of transfection.

21. Method according to claim 20, wherein said transfection is performed by electroporation, calciumphosphate co-precipitation, lipofection, the DEAE dextran technique or by retroviral gene transfer.

22. Method according to one or more of claims 18 - 21, wherein the selection is carried out in a medium containing mycophenolic acid, G418, or hygromycin as a selective agent.

23. Method according to one or more claims 18 - 22, wherein following introduction of a vector according to one or more claims 1 - 17 by homologous recombination a site-specific integration of said vector 3' of the heavy chain V gene of the malignant B cell is performed.

24. Method according to one or more claims 18 - 23, wherein the expression is controlled by the endogenous V_{H} promoter.

25. Use of a vector according to one or more claims 1 - 17 in the expression of immunoglobulin-cytokin fusion proteins in malignant B cells.

26. Use according to claim 25, wherein the malignant B cell is a B cell leukemia cell, a B cell lymphoma cell or a plasmacytoma cell.

27. Use according to claim 25, wherein by expression of the immunoglobulin-cytokin fusion proteins the activation of T cells is achieved.

28. Use of a vector according to one or more of claims 1 - 17 in malignant B cells for the vaccination of patients having malignant B cell diseases.

29. Malignant B cell containing a vector according to one or more of claims 1 - 17 in integrated form, wherein an immunoglobulin-cytokin fusion protein is expressed by said cell.

30. Use of a malignant B cell which has been rendered replication-incompetent and contains a vector according to one or more of claims 1 - 17 in integrated form and is capable of expression of an immunoglobulin-cytokin fusion protein for the preparation of a medicament in the treatment of patients having malignant B cell diseases.

## Revendications

1. Vecteur pour l'expression des protéines de fusion immunoglobuline cytokine dans des lymphocytes B malins, contenant au moins, dans des groupements fonctionnels :
(a) une région d'au moins 1,5 kb, qui est homologue d'une région de l'intron µ ou de l'intron κ ;
(b) au moins une séquence d'ADN, qui code un domaine d'une immunoglobuline ou une partie de domaine d'immunoglobuline ;
(c) une séquence d'ADN codant une cytokine ; et
(d) un gène marqueur sélectionnable dans des lymphocytes B eucaryotes, qui présente une région enhancer fonctionnelle, une région enhancer non fonctionnelle ou qui ne présente pas de région enhancer.

2. Vecteur selon la revendication 1, dans lequel la région d'au moins 1,5 kb contient un enhancer fonctionnel C_{µ} ou C_{κ}.

3. Vecteur selon la revendication 1, dans lequel la région d'au moins 1,5 kb contient un enhancer non fonctionnel C_{µ} ou C_{κ}.

4. Vecteur selon une ou plusieurs des revendications ci-dessus dans lequel le gène marqueur sélectionnable dans les lymphocytes B eucaryotes présente une région enhancer non fonctionnelle.

5. Vecteur selon une ou plusieurs des revendications ci-dessus, dans lequel le gène marqueur sélectionnable dans les lymphocytes B eucaryotes ne présente pas de région enhancer.

6. Vecteur selon une ou plusieurs des revendications ci-dessus, dans lequel la séquence d'ADN de (b) code une région constante ou une partie de région constante.

7. Vecteur selon une ou plusieurs des revendications ci-dessus, dans lequel la région qui est homologue à l'une des régions comprenant l'enhancer C_{µ} ou C_{κ} de l'intron µ ou κ est d'au moins 1,9 kb.

8. Vecteur selon une ou plusieurs des revendications ci-dessus, dans lequel la région qui est homologue à l'une des régions comprenant l'enhancer C_{µ} ou C_{κ} de l'intron µ ou κ est d' au moins 2,0 kb.

9. Vecteur selon une ou plusieurs des revendications ci-dessus, qui contient une unité de régulation compatible avec des bactéries.

10. Vecteur selon une ou plusieurs des revendications ci-dessus, dans lequel l'immunoglobuline est une immunoglobuline chimérique.

11. Vecteur selon une ou plusieurs des revendications ci-dessus, dans lequel la séquence d'ADN de (b) code le domaine d'une chaîne d'immunoglobuline humaine.

12. Vecteur selon une ou plusieurs des revendications ci-dessus dans lequel la séquence d'ADN de (b) code des domaines, qui proviennent de la souris, du rat, de la chèvre, du cheval ou du mouton.

13. Vecteur selon une ou plusieurs des revendications ci-dessus, dans lequel la séquence d'ADN de (b) code l'ensemble des domaines C d'un anticorps de sécrétion.

14. Vecteur selon une ou plusieurs des revendications ci-dessus, dans lequel la séquence d'ADN de (b) code l'ensemble des domaines C d'un anticorps lié à une membrane.

15. Vecteur selon une ou plusieurs des revendications ci-dessus, **caractérisé en ce que**, la séquence d'ADN de (c) code des interleukines, des interférons, des facteurs de stimulation de colonies, des lymphokines ou des facteurs de croissance.

16. Vecteur selon la revendication 15, **caractérisé en ce que**, la séquence d'ADN de (c) code l'IL-2, l'IL-4, l'IL-7, l'IL-12, l'IL-13, le GM-CSF ou l'interféron γ.

17. Vecteur selon une ou plusieurs des revendications ci-dessus, dans lequel le marqueur sélectionnable est gpt, neo ou un marqueur codant une résistance à l'hygromycine.

18. Procédé pour l'expression d'une protéine de fusion immunoglobuline - cytokine dans des lymphocytes B malins *in vitro* avec les étapes suivantes :
(a) introduction d'un vecteur selon une ou plusieurs des revendications 1 à 17 dans un lymphocyte B malin ;
(b) sélection et identification de cellules qui expriment la protéine de fusion de manière stable ;
(c) traitement des cellules pour qu'elles ne puissent pas se répliquer.

19. Procédé selon la revendication 18, dans lequel l'étape (b) est absente.

20. Procédé selon la revendication 18 ou 19, dans lequel l'étape (a) est réalisée par transfection.

21. Procédé selon la revendication 20, dans lequel la transfection est réalisée par électroporation, co-précipitation au phosphate de calcium, lipofection, la méthode dextran DEAE - ou transfert de gène par un vecteur rétroviral.

22. Procédé selon une ou plusieurs des revendications 18 à 21, dans lequel la sélection a lieu dans un milieu qui contient comme moyen de sélection de l'acide mycophénolique, du G418 ou de l'hygromycine.

23. Procédé selon une ou plusieurs des revendications 18 à 22, dans lequel après l'introduction d'un vecteur selon une ou plusieurs des revendications 1 à 17 par recombinaison homologue on réalise une intégration spécifique de site du vecteur en 3' du gène V de la chaîne lourde du lymphocyte B malin.

24. Procédé selon une ou plusieurs des revendications 18 à 23, dans lequel l'expression du promoteur V_{H} endogène est guidée.

25. Utilisation d'un vecteur selon une ou plusieurs des revendications 1 à 17 pour l'expression de protéines de fusion immunoglobuline - cytokine dans des lymphocytes B malins.

26. Utilisation selon la revendication 25, dans laquelle le lymphocyte B malin est une cellule de leucémie à lymphocyte B, une cellule de lymphome à lymphocyte B ou une cellule plasmocytome.

27. Utilisation selon la revendication 25, dans laquelle une activation des lymphocytes T est provoquée par l'expression de la protéine de fusion immunoglobuline - cytokine.

28. Utilisation d'un vecteur selon une ou plusieurs des revendications 1 à 17, dans des lymphocytes B malins pour la vaccination de patients atteints d'affections malignes à lymphocytes B.

29. Lymphocytes B malins, contenant un vecteur selon une ou plusieurs des revendications 1 à 17 sous forme intégrée, dans lesquels une protéine de fusion immunoglobuline - cytokine est exprimée par les lymphocytes.

30. Utilisation d'un lymphocyte B malin, qui a été rendu incapable de se réplicer et qui contient un vecteur selon une ou plusieurs des revendications 1 à 17 sous forme intégrée et qui est capable d'exprimer une protéine de fusion immunoglobuline - cytokine, pour la fabrication d'une composition pharmaceutique pour le traitement de patients atteints d'affections malignes à lymphocytes B.
